# EUROPEAN PATENT APPLICATION

(11) **EP 3 943 059 A1**
(43) Date of publication of application: **26.01.2022**
(21) Application number: 20773565.5
(22) Date of filing: 07.02.2020
(51) Int. Cl.: A61J 1/05, A61J 1/10, A61M 1/02

(54) **BLOOD BAG SYSTEM**

(30) Priority: 19.03.2019 JP 2019050789
(71) Applicant: TERUMO Kabushiki Kaisha, Tokyo 151-0072 (JP)
(72) Inventor: AGEISHI, Aya, Tokyo 163-1450 (JP); MARUTA, Chiaki, Tokyo 163-1450 (JP)
(74) Representative: TBK
(86) International application number: PCT/JP2020/004800
(87) International publication number: WO 2020/189083

(57) **Abstract**

A blood bag system (10) includes a blood bag (22) having a storage space (22a) for storing blood, a first tube (34) that is connected to the blood bag (22) and has a first flow path (34a) for communicating with the storage space (22a), and a sealing member (40) that is provided in the first tube (34) and opens the first flow path (34a) by a breaking action. The first tube (34) includes, at a position near the sealing member (40), an engagement portion (108) for engaging with a centrifugal transport device (12) with the sealing member (40) disposed at the position where the breaking action is performed.

## Description

### Technical Field

The present invention relates to a blood bag system set in a centrifugal transport device.

### Background Art

A blood bag system, which has a blood bag storing blood, is set in a centrifugal transport device at the time of centrifuging blood. The centrifugal transport device applies a centrifugal force to the set blood bag to separate the blood in the blood bag into a plurality of types of blood components, and transports a predetermined blood component to another bag via a tube connected to the blood bag.

Such a type of blood bag system includes a sealing member (communicating piece) inside a tube connecting a blood bag and another bag (see JP H6-197955 A). The sealing member blocks outflow of blood from the blood bag when blood of a donor is stored in the blood bag. Then, when a user sets the blood bag system in a centrifugal transport device, the sealing member is disposed in a breaking portion provided in the centrifugal transport device and is broken by the breaking portion so that the blood component can be transported from the blood bag.

### Summary of Invention

Meanwhile, when the blood bag is set in the centrifugal transport device, the sealing member is pulled by the blood bag having blood (weight), so that the sealing member is sometimes displaced with respect to the breaking portion. When the sealing member is disposed so as to be displaced from the breaking portion, it is difficult for the breaking portion to break the sealing member. In particular, the sealing member in the tube of the blood bag system is hardly visually recognized, there is a possibility that the user does not notice even if the sealing member is displaced.

The present invention has been made in relation to the above-described technique of setting a blood bag system in a centrifugal transport device, and an object thereof is to provide a blood bag system capable of reliably breaking a sealing member by maintaining a positioning state of the sealing member with a simple configuration.

In order to achieve the above object, an aspect of the present invention is a blood bag system including: a blood bag having a storage space for storing blood; a tube that is connected to the blood bag and has a flow path for communicating with the storage space; and a sealing member that is provided in the tube and opens the flow path by a breaking action. The tube includes, at a position near the sealing member, an engagement portion for engaging with a centrifugal transport device with the sealing member disposed at a position where the breaking action is performed.

In the above-described blood bag system, the sealing member can be firmly positioned with respect to the position where the breaking action of the centrifugal transport device is performed, with the simple configuration in which the tube includes the engagement portion. That is, even if the blood bag pulls the sealing member by its weight, the movement of the sealing member can be suppressed by the engagement portion engaged with the centrifugal transport device. Therefore, the positioning state of the sealing member can be favorably maintained in the blood bag system, and the sealing member can be reliably broken.

### Brief Description of Drawings

Fig. 1 is an explanatory view illustrating an overall configuration of a blood bag system and a centrifugal transport device according to an embodiment of the present invention.
Fig. 2 is a plan view illustrating a unit set area of the centrifugal transport device.
Fig. 3 is a front view illustrating a blood bag.
Fig. 4 is a perspective view illustrating a state where the blood bag is set in the unit set area.
Fig. 5 is an enlarged plan view illustrating an engagement structure in a state of being set in the centrifugal transport device.
Fig. 6 is an enlarged plan view illustrating an engagement structure according to a first modification.
Fig. 7 is an enlarged plan view illustrating an engagement structure according to a second modification.
Fig. 8 is an enlarged plan view illustrating an engagement structure according to a third modification.

### Description of Embodiments

Hereinafter, preferred embodiments of the present invention will be described in detail with reference to the accompanying drawings.

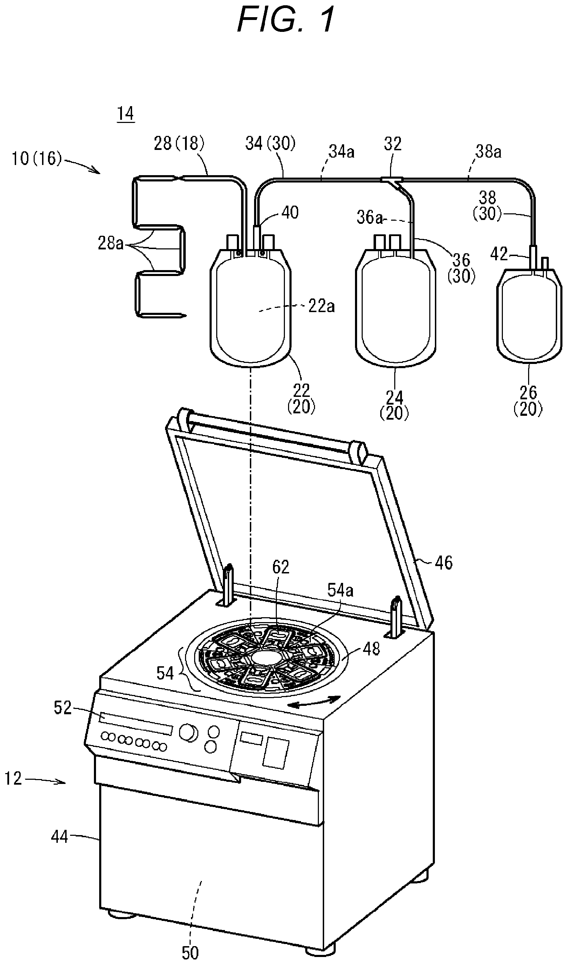

A blood bag system 10 according to an embodiment of the present invention is set in a centrifugal transport device 12 as illustrated in Fig. 1 by a user such as a medical worker. The centrifugal transport device 12 centrifuges blood in the blood bag system 10 to generate and store a plurality of types of blood components. Hereinafter, a configuration including the blood bag system 10 and the centrifugal transport device 12 is referred to as a blood product manufacturing system 14.

The blood bag system 10 includes a pretreatment unit (not illustrated) used for blood sampling before centrifugation and a separator unit 16 that generates blood components by centrifugation and individually stores the blood components, and the both units are connected by a relay tube 18. The separator unit 16 is set in the centrifugal transport device 12 in a state where the relay tube 18 connected to the pretreatment unit is cut into the state illustrated in Fig. 1 before the centrifugation.

The pretreatment unit of the blood bag system 10 collects whole blood from a donor (blood donor) and removes a predetermined blood component (for example, a white blood cell) from the whole blood. Therefore, the pretreatment unit includes a blood collection needle, a blood collection bag, an initial flow blood bag, a filter (for example, a white blood cell removal filter), and the like, and is configured by connecting the respective members via a plurality of tubes. The separator unit 16 is connected to the downstream side of the filter via the relay tube 18. Specifically, the pretreatment unit stores the first blood out of the whole blood of the donor, collected through the blood collection needle, in the initial flow blood bag, and then stores the remaining blood in the blood collection bag. Further, the pretreatment unit removes white blood cells in the filter by causing the whole blood, stored in the blood collection bag, to flow to the filter, and transports the removed blood (white-blood-cell-removed blood) to the separator unit 16.

On the other hand, the separator unit 16 of the blood bag system 10 includes a plurality of bags 20 (a blood bag 22, a PPP bag 24, and a liquid drug bag 26), and is configured by connecting the respective bags 20 via a plurality of tubes 30.

The blood bag 22 is directly connected to the relay tube 18 connected to the pretreatment unit in a state of the blood bag system 10 provided as a product. Therefore, the blood bag 22 stores the removed blood transported from the filter at the time of collecting blood. The blood bag 22 is set in the centrifugal transport device 12, and a centrifugal force is applied by the operation of the centrifugal transport device 12. As a result, the removed blood in the blood bag 22 is centrifuged into blood components (platelet poor plasma (PPP), red blood cells (RBC)) and the like having different specific gravities. Then, the blood bag 22 transports PPP under the operation of the centrifugal transport device 12 after centrifugation to store only the remaining RBC.

The PPP bag 24 stores PPP by receiving the PPP supplied from the blood bag 22. On the other hand, the liquid drug bag 26 contains a red blood cell preservation solution, such as a MAP solution, an SAGM solution, and OPTISOL, in advance.

In addition, a segment tube 28 is formed before the separator unit 16 is set in the centrifugal transport device 12. The segment tube 28 has a plurality of sealed tubes 28a in which blood of the donor (removed blood) exists. The plurality of sealed tubes 28a are formed so as to be continuous in series by cutting the relay tube 18, which connects the blood bag 22 and the pretreatment unit (filter), near the filter and further sealing the cut tube at predetermined intervals. The segment tube 28 is cut by a user as necessary, and is used for checking blood or the like.

The plurality of tubes 30 of the separator unit 16 branch into a plurality of tubes via a branching connector 32 (Y-type connector). Specifically, the plurality of tubes 30 include a first tube 34 connecting the blood bag 22 and the branching connector 32, a second tube 36 connecting the PPP bag 24 and the branching connector 32, and a third tube 38 connecting the liquid drug bag 26 and the branching connector 32. The first tube 34 has a first flow path 34a, the second tube 36 has a second flow path 36a, and the third tube 38 has a third flow path 38a. The first to third flow paths 34a, 36a, and 38a communicate with each other via a flow path in the branching connector 32.

A breakable sealing member 40 (click tip) is provided at an end portion of the first tube 34 on the blood bag 22 side. Similarly, a breakable sealing member 42 is provided at an end portion of the third tube 38 on the liquid drug bag 26 side. The sealing members 40 and 42 block the first flow path 34a and the third flow path 38a until a breaking operation is performed, and prevent a liquid in the blood bag 22 and the liquid drug bag 26 from being transported to the other bag 20.

On the other hand, the centrifugal transport device 12 in which the separator unit 16 of the blood bag system 10 is set includes a box-shaped base body 44, a lid 46 capable of opening and closing an upper surface of the base body 44, and a centrifugal drum 48 provided on the base body 44. In addition, the base body 44 of the centrifugal transport device 12 is provided with a motor (not illustrated) that rotates the centrifugal drum 48, a control unit 50 that controls the operation of the centrifugal transport device 12, and an operation display unit 52 configured to allow the user to perform confirmation and operation.

The centrifugal drum 48 has a plurality of (six) unit set areas 54 in which the separator unit 16 can be set. A height of one unit set area 54 is longer than a longitudinal length of the bag 20, and is set in a range of 60° with respect to a rotation center of the centrifugal drum 48. That is, the six unit set areas 54 are arrayed without a gap along the circumferential direction to form the centrifugal drum 48 which is an annular structure.

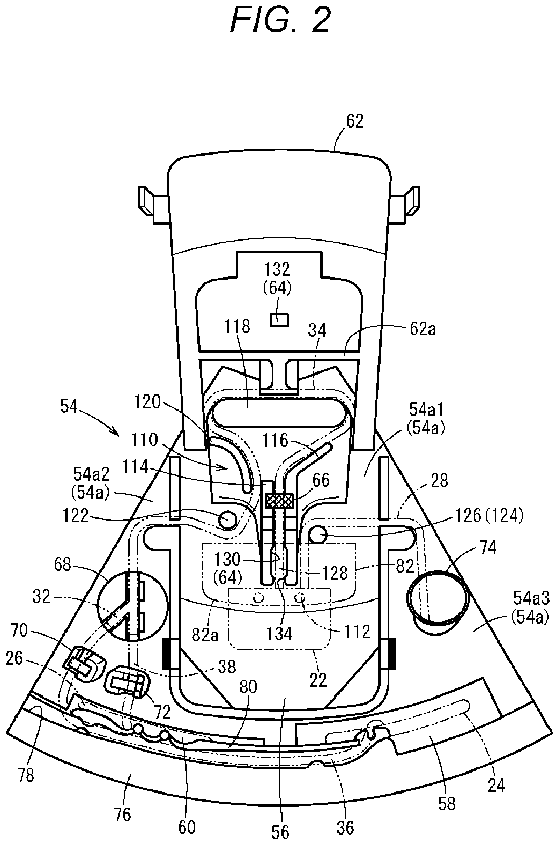

As illustrated in Figs. 1 and 2, the unit set area 54 applies the centrifugal force to the blood bag 22 as the centrifugal drum 48 rotates. Specifically, the unit set area 54 includes a blood bag pocket 56 accommodating the blood bag 22, a PPP bag pocket 58 accommodating a PPP bag 24, and a liquid drug bag pocket 60 accommodating the liquid drug bag 26 at radially outer positions of the centrifugal drum 48.

The blood bag pocket 56 is provided at the circumferential center of the unit set area 54 and has a larger volume than the PPP bag pocket 58 or the liquid drug bag pocket 60. The PPP bag pocket 58 and the liquid drug bag pocket 60 are provided side by side in the circumferential direction on the radially outer side of the blood bag pocket 56.

In addition, an upper surface 54a of the unit set area 54 is configured such that the plurality of tubes 30 of the blood bag system 10 are disposed and retained thereon. The first tube 34 and a part of the segment tube 28 are disposed in a central region 54a1 (first region) on the radially inner side of the blood bag pocket 56 on the upper surface 54a. The central region 54a1 is provided with a lid body 62 that opens and closes an opening of the blood bag pocket 56. Further, a sensor 66 (optical sensor), which detects a part of a breaking portion 64 that breaks the sealing member 40 and a state of blood flowing through the first tube 34, is provided at an arrangement position of the first tube 34 closed by the lid body 62.

A part of the first tube 34 passing through the central region 54a1, the branching connector 32, a part of the second tube 36, and a part of the third tube 38 are disposed in a left region 54a2 (second region) of the upper surface 54a. The left region 54a2 is provided with a holder 68 that retains the branching connector 32, a PPP clamp 70 that opens and closes the second flow path 36a of the second tube 36, and a liquid drug clamp 72 that opens and closes the third flow path 38a of the third tube 38.

A part of the segment tube 28 passing through the central region 54a1 is disposed in a right region 54a3 (third region) of the upper surface 54a. The right region 54a3 is provided with a segment pocket 74 that accommodates the plurality of sealed tubes 28a of the segment tube 28.

Further, a tube retaining portion 76 protruding upward from the upper surface 54a is provided on the radially outer side of the PPP bag pocket 58 and the liquid drug bag pocket 60 of the unit set area 54. The tube retaining portion 76 has an outer wall 78 continuous with an outer peripheral edge of the unit set area 54 and an inner wall 80 protruding to the inner side of the outer wall 78 from the outer peripheral edge, and the second tube 36 is disposed between the outer wall 78 and the inner wall 80. The inner wall 80 extends from the vicinity of the PPP clamp 70 in the left region 54a2 (left side of the liquid drug bag pocket 60) to a circumferential intermediate position of the PPP bag pocket 58, and guides the second tube 36 to the PPP bag pocket 58.

In addition, the unit set area 54 includes a slider 82 (pusher), which presses the blood bag 22 after centrifugation, on the radially inner side of the blood bag pocket 56. A surface of the slider 82 facing the blood bag 22 is formed on an inclined surface 82a. The slider 82 moves forward and backward along the radial direction of the centrifugal drum 48 under the control of the control unit 50 (see Fig. 1).

In the unit set area 54, the user accommodates the blood bag 22 storing the removed blood, the empty PPP bag 24, and the liquid drug bag 26 storing the liquid drug in the blood bag pocket 56, the PPP bag pocket 58, and the liquid drug bag pocket 60, respectively. Then, the unit set area 54 centrifuges the removed blood in the blood bag 22 by the rotation of the centrifugal drum 48, advances the slider 82 after the centrifugation to press the blood bag 22. As a result, PPP generated by the centrifugation in the blood bag 22 is transported to the PPP bag 24, and RBC remains in the blood bag 22 after the transport of PPP. Thereafter, the blood bag system 10 is taken out from the centrifugal transport device 12 by the user, and the liquid drug bag 26 is hung on a stand (not illustrated). As a result, the red blood cell preservation solution is supplied from the liquid drug bag 26 to the blood bag 22, and the blood bag 22 is turned into a state of storing the RBC (blood products) containing a liquid drug.

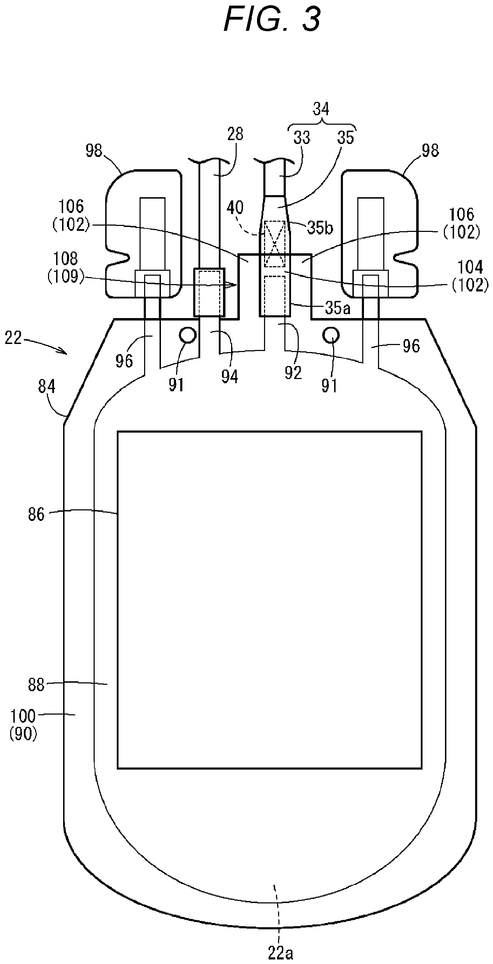

Next, the blood bag 22, which is a main part of the blood bag system 10 according to the present embodiment, the first tube 34 connected to the blood bag 22, and the like will be described with reference to Fig. 3.

The blood bag 22 includes a bag main body 84 having a storage space 22a capable of storing blood (removed blood). The bag main body 84 is formed in a bag shape by overlapping sheets to seal the periphery. In addition, a label 86 on which predetermined information has been printed is attached to the outer surface of the bag main body 84.

Specifically, the bag main body 84 includes an accommodating portion 88 forming the storage space 22a and a seal part 90 which is a portion sealed around the accommodating portion 88 in plan view (front view seen from the label 86 side). A plurality (one set) of bag holes 91 are provided in the seal part 90 at one end portion (upper portion) in the longitudinal direction of the bag main body 84. When the blood bag system 10 is set in the centrifugal transport device 12, pins 112 and the like of the unit set area 54 are inserted into the plurality of bag holes 91, so that the blood bag 22 is turned into a suspended state.

In addition, a first connection port 92 to which the first tube 34 is connected, a second connection port 94 to which the segment tube 28 is connected, and two extraction ports 96 are provided in the upper portion of the bag main body 84. Incidentally, the extraction port 96 is covered with a cover 98 that blocks outflow of blood.

Each of the ports 92, 94, and 96 is welded in the state of communicating with the storage space 22a at the time of forming the seal part 90. The first and second connection ports 92 and 94 are firmly fixed to end portions of the tubes 28 and 34 by an appropriate fixing means such as welding and adhesion. The first tube 34 includes an elongated tube main body 33 forming most of its entire length, and a cylindrical connecting end portion 35 that is continuous with an end portion of the tube main body 33 and is formed thick to be connected to the first connection port 92.

The connecting end portion 35 includes a large-diameter portion 35a connected to the first connection port 92 and a tapered portion 35b that is connected to the large-diameter portion 35a and is reduced in diameter toward the tube main body 33. The above-described sealing member 40 is provided inside the connecting end portion 35. The sealing member 40 is disposed at a position near the downstream side of the first connection port 92 and extends across the large-diameter portion 35a and the tapered portion 35b along the axial direction of the first tube 34.

In addition, the large-diameter portion 35a of the connecting end portion 35 is a portion sealed by the seal part 90 of the bag main body 84. That is, the seal part 90 has a connecting seal region 102 that seals a position corresponding to the connecting end portion 35 in addition to the seal main body 100 that seals the periphery of the accommodating portion 88 in plan view of the bag main body 84.

The connecting seal region 102 is continuous with the seal main body 100 as pieces protruding from a pair of sheets forming the seal main body 100 are sealed together with the formation of the seal part 90. The connecting seal region 102 formed in this manner has a sealed portion 104 that is directly sealed to the outer peripheral surface of the connecting end portion 35 (large-diameter portion 35a) and a pair of continuous pieces 106 protruding to both sides in the width direction of the sealed portion 104.

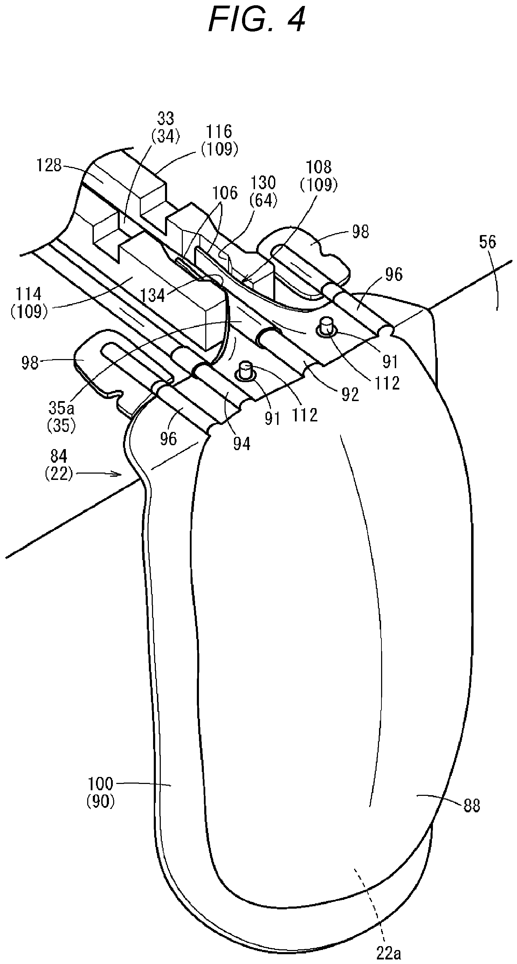

The pair of continuous pieces 106 is formed by sealing the pair of sheets as described above, and thus, has flexibility softer than the first tube 34 and a certain degree of thickness. The pair of continuous pieces 106 functions as an engagement portion 108 that reinforces positioning of the sealing member 40 (connecting end portion 35) when the blood bag 22 and the first tube 34 are set in the centrifugal transport device 12. That is, the continuous piece 106 (engagement portion 108) forms an engagement structure 109 together with a first tube guide portion 110 provided on the upper surface 54a (central region 54a1) of the unit set area 54 as illustrated in Fig. 4.

Specifically, the central region 54a1 has the plurality (a set) of pins 112 on the radially inner side of the blood bag pocket 56, and includes the first tube guide portion 110 on the radially inner side of the pins 112. As illustrated in Fig. 2, the first tube guide portion 110 includes: a pair of ridge portions 114 and 116 extending along the radial direction of the unit set area 54; a folded portion 118 extending in the width direction on the radially inner side; a curved portion 120 that is curved and extends on the left side of the ridge portion 114 and on the radially outer side of the folded portion 118; and a left guide portion 122 formed to protrude on the left side of the ridge portion 114 and on the radially outer side of the curved portion 120. Incidentally, a right guide portion 126 forming the segment tube guide portion 124 is provided on the right side of the ridge portion 116.

The pair of ridge portions 114 and 116 forms an arrangement passage 128 in which the first tube 34 is disposed along the radial direction. In addition, the ridge portion 116 extends to be longer than the ridge portion 114 in order to guide the first tube 34 to the folded portion 118, and a radially inner portion of the ridge portion 116 is inclined. The arrangement passage 128 is provided with a part of the breaking portion 64 that breaks the sealing member 40 of the connecting end portion 35 and the sensor 66.

The breaking portion 64 includes: a wide space 130 in which the arrangement passage 128 is widened in the width direction by slightly notching inner side surfaces of the pair of ridge portions 114 and 116; and a compression portion 132 that is provided on a back surface of the lid body 62 and protrudes by a predetermined height. The sealing member 40 of the connecting end portion 35 is just disposed in the wide space 130 when the blood bag 22 is suspended through the pin 112 in the bag hole 91 of the blood bag 22 and the first tube 34 is disposed in the arrangement passage 128. The compression portion 132 just enters the wide space 130 when the lid body 62 is rotated relative to the upper surface 54a to close the blood bag pocket 56. That is, when the lid body 62 is rotated after the blood bag system 10 is set in the unit set area 54, the compression portion 132 enters the wide space 130 and presses the sealing member 40 to break the sealing member 40.

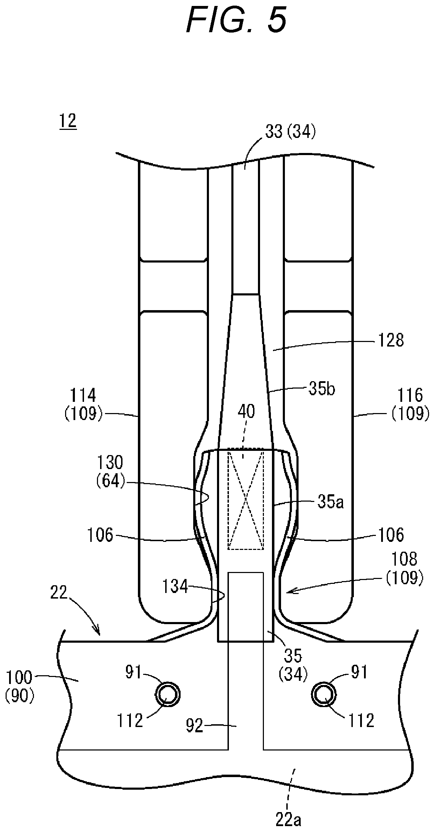

As illustrated in Figs. 4 and 5, radially outer ends of the pair of ridge portions 114 and 116 form a narrow space 134 in which a width of the arrangement passage 128 is narrower than that in the wide space 130. A width of the narrow space 134 is set to be, for example, substantially the same as a diameter of the connecting end portion 35 (large-diameter portion 35a) of the first tube 34. Therefore, the blood bag system 10 accommodates the pair of continuous pieces 106 as well as the connecting end portion 35 in the narrow space 134 while deforming the pair of continuous pieces 106 when the connecting end portion 35 is disposed in the narrow space 134.

In other words, the pair of ridge portions 114 and 116 forms the engagement structure 109 for positioning the sealing member 40 together with the pair of continuous pieces 106 (engagement portion 108). That is, the pair of continuous pieces 106 greatly increases a frictional force of the connecting end portion 35 with respect to the pair of ridge portions 114 and 116, thereby firmly fixing the connecting end portion 35.

The blood bag system 10 according to the present embodiment is basically configured as described above, and operations thereof will be described hereinafter.

As described above, the blood bag system 10 stores the removed blood, obtained by removing a predetermined component (white blood cell) from the whole blood of the donor, in the blood bag 22 by using the pretreatment unit at the time of collecting blood by the user (medical worker). In the storage space 22a, blood of an appropriate blood volume (for example, 400 cc) according to the donor is stored.

Thereafter, the user separates the separator unit 16 of the blood bag system 10 from the pretreatment unit and sets the separator unit 16 in the centrifugal transport device 12 in order to centrifuge the removed blood. The blood bag 22 is accommodated in the blood bag pocket 56 in a state where the pins 112 are inserted into the bag holes 91 of the unit set area 54. In addition, the first tube 34 is disposed along the first tube guide portion 110 on the upper surface 54a.

At this time, the first tube 34 close to the blood bag 22 is disposed in the arrangement passage 128 between the pair of ridge portions 114 and 116. Then, the connecting end portion 35 and the pair of continuous pieces 106 are accommodated in the narrow space 134 on the radially outer side of the arrangement passage 128. At this time, the connecting end portion 35 and the pair of continuous pieces 106 are engaged by receiving a large frictional force from the pair of ridge portions 114 and 116.

Here, in a case where the blood bag 22 is heavy, a central portion of the blood bag 22 along an axial center of the first tube 34 is pulled downward even if the bag hole 91 is caught by the pin 112. Therefore, the blood bag 22 is pulled toward the blood bag pocket 56 with respect to the first tube 34 (connecting end portion 35). In the present embodiment, however, the movement of the connecting end portion 35 is suppressed by the pair of continuous pieces 106 serving as the engagement portion 108 as described above. That is, the sealing member 40 in the connecting end portion 35 can be accurately disposed in the breaking portion 64 (wide space 130). Therefore, when the user closes the lid body 62, the compression portion 132 of the breaking portion 64 reliably breaks the sealing member 40 to open the first flow path 34a of the first tube 34.

After the blood bag system 10 is set, the centrifugal transport device 12 centrifuges the removed blood in the blood bag 22 into blood components having different specific gravities (PPP, RBC, and the like) by rotating the centrifugal drum 48 under the control of the control unit 50. After such centrifugation, the centrifugal transport device 12 presses the blood bag 22 by the slider 82. As a result, PPP having a light specific gravity flows out from the blood bag 22, and the PPP flows through the first tube 34, the branching connector 32, and the second tube 36 in this order and flows into the PPP bag 24. Since the sealing member 40 reliably opens the first flow path 34a as described above, the transport of PPP is not prevented.

When PPP is transported from the blood bag 22 to the PPP bag 24, the centrifugal transport device 12 retracts the slider 82 so that the blood bag 22 can be taken out from the blood bag pocket 56. At this time, the deformed pair of continuous pieces 106 project from the pair of ridge portions 114 and 116. The user can easily release the engagement of the engagement portion 108 with the first tube guide portion 110 by pulling the continuous piece 106.

Thereafter, the user takes out the blood bag system 10 from the centrifugal transport device 12, and suspends the liquid drug bag 26 on the stand to supply the red blood cell preservation solution to the blood bag 22. As a result, RBC containing the red blood cell preservation solution is stored in the blood bag 22.

Incidentally, the present invention is not limited to the above-described embodiment, and various modifications can be made in accordance with a gist of the invention. For example, the plurality (the pair) of continuous pieces 106 are formed as the engagement portion 108 on both sides of the connecting end portion 35 in the width direction in the above-described embodiment. However, only one continuous piece 106 may be provided.

Hereinafter, some several modifications according to the present invention will be described. Incidentally, an element having the same configuration or the same function as that of the above-described embodiment will be denoted by the same reference sign, and the detailed description thereof will be omitted in the following description.

### [First Modification]

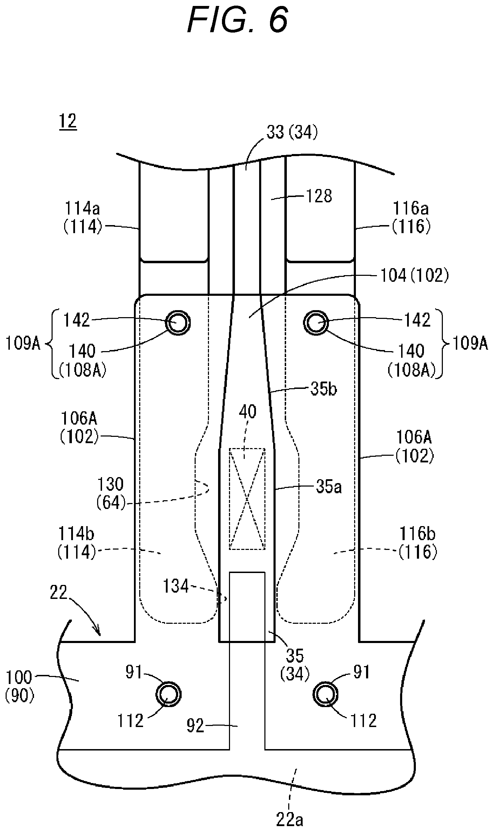

As illustrated in Fig. 6, an engagement structure 109A according to a first modification includes: positioning holes 140 (engagement portions 108A) of continuous pieces 106A of the blood bag system 10; and engaged pins 142 of the centrifugal transport device 12. That is, the engagement structure 109A performs positioning of the sealing member 40 by passing the engaged pins 142 through the positioning holes 140 to hook the continuous pieces 106A.

Specifically, the centrifugal transport device 12 includes the engaged pin 142 on each of the pair of ridge portions 114 and 116 provided on the centrifugal drum 48 (unit set area 54). In this case, the pair of ridge portions 114 and 116 protrude from the upper surface 54a, but are formed in a stepped shape such that radially outer portions 114b and 116b are lower than radially inner portions 114a and 116a. Each of the radially outer portions 114b and 116b has a trapezoidal shape in which the pair of continuous pieces 106A can be disposed. In the radially outer portions 114b and 116b, the engaged pins 142 are provided at positions on the radially inner side of the wide space 130. Incidentally, the continuous piece 106A is engaged on the radially inner side of the breaking portion 64 in the present modification, and thus, shapes and the like of the ridge portions 114 and 116 on the radially outer side of the engagement portion are not particularly limited. For example, it may be configured such that the arrangement passage 128 extends without providing the narrow space 134 formed by the pair of ridge portions 114 and 116 (or by forming the narrow space 134 to be somewhat wide). Alternatively, the radially outer portions 114b and 116b of the pair of ridge portions 114 and 116 are not necessarily provided.

On the other hand, the connecting seal region 102 of the seal part 90 is formed over the entire length of the connecting end portion 35 of the first tube 34, and includes the pair of continuous pieces 106A extending to be long on both sides in the width direction of the connecting end portion 35. The positioning hole 140 is formed in the vicinity of an extending end portion of each of the continuous pieces 106A. That is, the pair of positioning holes 140 is provided at positions protruding to the inner side in the centrifugal direction from the sealing member 40 (positions away from the seal main body 100) in a state where the blood bag 22 is suspended.

In the engagement structure 109A configured as described above, the positioning holes 140 are engaged with the pair of engaged pins 142 on the inner side in the centrifugal direction with respect to the sealing member 40 in a state where the connecting end portion 35 is disposed in the arrangement passage 128 between the pair of ridge portions 114 and 116. Therefore, the blood bag 22 hooks the pair of pins 112 on the pair of bag holes 91 and hooks the pair of engaged pins 142 on the pair of positioning holes 140. That is, since the engagement structure 109A is engaged on the radially inner side of the sealing member 40, the sealing member 40 is suppressed from moving to the radially outer side even if the blood bag 22 accommodated in the blood bag pocket 56 pulls the sealing member 40.

Incidentally, it suffices that a position of the engagement structure 109A (the positioning hole 140 and the engaged pin 142) is a position farther than the pair of bag holes 91 with respect to the storage space 22a in which blood is stored. For example, the engagement structure 109A may include the positioning hole 140 in the continuous piece 106A at a position with the same height as the sealing member 40. In addition, the engaged pin 142 may be provided at an appropriate position according to the position of the positioning hole 140.

### [Second Modification]

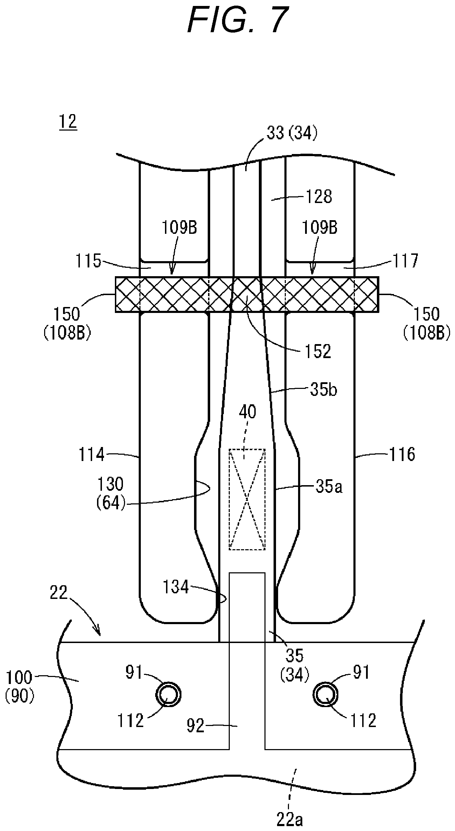

As illustrated in Fig. 7, an engagement structure 109B according to a second modification includes: protruding portions 150 (engagement portions 108B) provided at the connecting end portion 35 of the first tube 34; and grooves 115 and 117 provided in advance in the pair of ridge portions 114 and 116 of the centrifugal transport device 12.

Specifically, the pair of ridge portions 114 and 116 provided in the unit set area 54 has the grooves 115 and 117 cut out along the width direction of the ridge portions 114 and 116 at intermediate positions in the extending direction. These grooves 115 and 117 are formed in advance in order to dispose an outer edge protrusion 62a (see Fig. 2) provided inside the breaking portion 64 (compression portion 132) in the lid body 62. When the blood bag pocket 56 is closed by the lid body 62, the outer edge protrusion 62a enters the grooves 115 and 117, so that the grooves 115 and 117 suppress lift of the first tube 34 (connecting end portion 35) sandwiched between the pair of ridge portions 114 and 116.

The pair of protruding portions 150 of the blood bag system 10 protrudes from a connection part 152 to both sides in the width direction. In addition, the pair of protruding portions 150 extends parallel to the seal part 90 of the blood bag 22 and protrudes to be longer than widths of the ridge portions 114 and 116. Each of the protruding portions 150 is harder than the first tube 34.

In addition, the connection part 152 is connected to an upper end (the inner side in the centrifugal direction) of the connecting end portion 35. That is, the protruding portions 150 are provided at positions protruding to the inner side in the centrifugal direction from the sealing member 40 (positions away from the seal main body 100) in a state where the blood bag 22 is suspended.

In the engagement structure 109B configured as described above, the protruding portions 150 are disposed in the grooves 115 and 117 on the radially inner side of the sealing member 40 in a state where the connecting end portion 35 is disposed in the arrangement passage 128 between the pair of ridge portions 114 and 116. Therefore, the engagement structure 109B is engaged on the radially inner side of the sealing member 40, and the sealing member 40 is suppressed from moving to the radially outer side even if the blood bag 22 accommodated in the blood bag pocket 56 pulls the sealing member 40.

### [Third Modification]

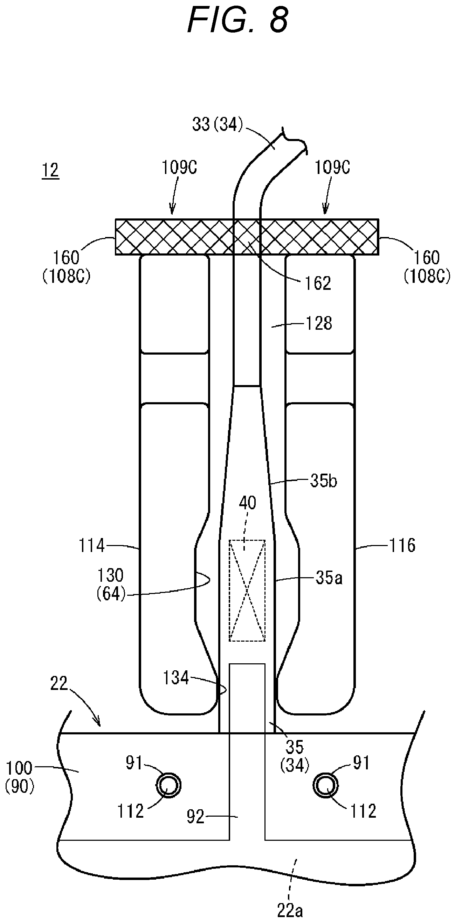

As illustrated in Fig. 8, an engagement structure 109C according to a third modification includes: protruding portions 160 (engagement portions 108C) provided in the first tube 34 of the blood bag system 10; and the ridge portions 114 and 116 of the centrifugal transport device 12. The protruding portions 160 are configured to be hard similarly to the protruding portions 150 according to the second modification, and protrude from a connection part 162 to both sides in the width direction. The connection part 162 is firmly fixed to an outer peripheral surface of the first tube 34 at a predetermined position. That is, the engagement structure 109C includes the pair of protruding portions 160 at positions corresponding to ends on the centrifugal center side of the ridge portions 114 and 116 in the middle of the extension of the first tube 34. As a result, the protruding portions 160 can be engaged with the ridge portions 114 and 116 at the time of setting the blood bag 22.

In short, a position where the engagement structure 109C (engagement portions 108C) is provided may be the first tube 34 as long as the position is relatively close to the blood bag 22. In addition, a structure on the centrifugal transport device 12 side with which the engagement portions 108 and 108A to 108C are engaged is not limited to the pair of ridge portions 114 and 116, and various configurations may be adopted as long as the engagement portions 108 and 108A to 108C can be engaged in a state where the first tube 34 is stretched.

Technical ideas and effects that can be grasped from the above-described embodiment are described as follows.

The blood bag system 10 includes the engagement portion 108 or 108A to 108C in the blood bag 22 or the tube 30 (the first tube 34), and thus, can firmly position the sealing member 40 with respect to the breaking portion 64 of the centrifugal transport device 12. That is, even if the blood bag 22 pulls the sealing member 40 by its weight, the centrifugal transport device 12 is engaged with the engagement portion 108 or 108A to 108C at the position farther from the storage space 22a than the bag hole 91, so that the movement of the sealing member 40 is suppressed. Therefore, the blood bag system 10 can favorably maintain the positioning state of the sealing member 40, and the sealing member 40 can be reliably broken under the breaking action of the breaking portion 64.

The tube 30 (first tube 34) includes the elongated tube main body 33 and the connecting end portion 35 that is connected to the connection port (first connection port 92) of the blood bag 22 at an end portion of the tube main body 33 and has the sealing member 40 inside. Since the blood bag system 10 suppresses the movement of the connecting end portion 35 along the axial direction by the engagement portion 108 or 108A to 108C, the sealing member 40 can be more accurately positioned with respect to the breaking portion 64.

The engagement portion 108 is the continuous piece 106 continuous with the sealed portion which seals the connecting end portion 35. As a result, the blood bag system 10 can easily position and fix the sealing member 40 only by a simple operation of accommodating the continuous piece 106 together with the connecting end portion 35 when being set in the centrifugal transport device 12.

The connecting end portion 35 includes the large-diameter portion 35a directly connected to the connection port (first connection port 92) and the tapered portion 35b that is continuous with the large-diameter portion 35a and tapered toward the tube main body 33, and the continuous piece 106 is continuous with the outer peripheral surface of the large-diameter portion 35a. As a result, the blood bag system 10 can accommodate the continuous piece 106 together with a thick portion of the connecting end portion 35 between the pair of ridge portions 114 and 116 to perform firm positioning.

In addition, the continuous piece 106A connected to the sealed portion which seals the connecting end portion 35 is provided, and the engagement portion 108A is the positioning hole 140 which is provided in the continuous piece 106A and into which the engaged pin 142 of the centrifugal transport device 12 is inserted. As a result, the blood bag system 10 can be engaged with the centrifugal transport device 12 only by passing the engaged pin 142 through the positioning hole 140 of the continuous piece 106A. Moreover, the continuous piece 106A is reliably prevented from moving by the engagement portion 108A, and thus, the sealing member 40 can be positioned more firmly.

In addition, the continuous piece 106A is formed over the entire length in the extending direction of the connecting end portion 35, and the positioning hole 140 is provided on the inner side in the centrifugal direction with respect to the sealing member 40. As a result, when the blood bag 22 pulls the sealing member 40, tension is applied from the blood bag 22 to the entire continuous piece 106A, so that the movement of the sealing member 40 can be suppressed by the entire continuous piece 106A.

In addition, the engagement portion 108B includes the protruding portion 150 that is connected to the connecting end portion 35 and protrudes to the outer side in the width direction of the connecting end portion 35. As a result, the protruding portion 150 can be engaged with the grooves 115 and 117 and the like provided in advance in the pair of ridge portions 114 and 116 in the blood bag system 10, and the sealing member 40 can be more easily positioned.

In addition, the engagement portion 108C includes the protruding portion 160 that is connected to the tube main body 33 and protrudes to the outer side in the width direction of the tube main body 33. In this manner, the blood bag system 10 can be engaged directly with the pair of ridge portions 114 and 116 by the protruding portion 160 provided on the tube 30, so that the sealing member 40 can be positioned more easily.

In addition, the protruding portions 150 and 160 are configured to be harder than the tube 30 (first tube 34). As a result, an engaging force between each of the protruding portions 150 and 160 and each of the ridge portions 114 and 116 is increased in the blood bag system 10, and thus, the sealing member 40 can be more firmly positioned.

## Claims

1. A blood bag system comprising:
a blood bag having a storage space for storing blood;
a tube that is connected to the blood bag and has a flow path for communicating with the storage space; and
a sealing member that is provided in the tube and opens the flow path by a breaking action,
wherein the tube includes, at a position near the sealing member, an engagement portion for engaging with a centrifugal transport device with the sealing member disposed at a position where the breaking action is performed.

2. The blood bag system according to claim 1, wherein
the tube includes an elongated tube main body and a connecting end portion that is connected to a connection port of the blood bag at an end portion of the tube main body and has the sealing member inside.

3. The blood bag system according to claim 2, wherein
the engagement portion is a continuous piece that is continuous with a sealed portion which seals the connecting end portion.

4. The blood bag system according to claim 3, wherein
the connecting end portion includes a large-diameter portion directly connected to the connection port and a tapered portion that is continuous with the large-diameter portion and tapered toward the tube main body, and
the continuous piece is continuous with an outer peripheral surface of the large-diameter portion.

5. The blood bag system according to claim 2, comprising
a continuous piece continuous with a sealed portion which seals the connecting end portion, wherein
the engagement portion is a positioning hole which is provided in the continuous piece and into which an engaged pin of the centrifugal transport device is inserted.

6. The blood bag system according to claim 5, wherein
the continuous piece is formed over a whole length of the connecting end portion in an extending direction, and
the positioning hole is provided on an inner side in a centrifugal direction with respect to the sealing member.

7. The blood bag system according to claim 2, wherein
the engagement portion includes a protruding portion that is connected to the connecting end portion and protrudes to an outer side in a width direction of the connecting end portion.

8. The blood bag system according to claim 2, wherein
the engagement portion includes a protruding portion that is connected to the tube main body and protrudes to an outer side in a width direction of the tube main body.

9. The blood bag system according to claim 7 or 8, wherein
the protruding portion is configured to be harder than the tube.
